# EUROPEAN PATENT APPLICATION

(11) **EP 2 179 750 A2**
(43) Date of publication of application: **28.04.2010**
(21) Application number: 09170109.4
(22) Date of filing: 11.09.2009
(51) Int. Cl.: A61L 27/54, A61L 29/16, A61L 31/16

(54) **Elastomeric devices containing chlorhexidine/fatty acid salts made from fatty acids of 12 to 18 carbons**

(30) Priority: 12.09.2008 US 96650 P
(71) Applicant: Arrow International, Inc., Reading, Pennsylvania 19605 (US)
(72) Inventor: Okoh, Onajite, Reading, PA 19602 (US); Do, Hiep, Sinking Spring, PA 19608 (US); Rosenblatt, Joel, Pottstown, PA 19465 (US)
(74) Representative: Stephen, Robert John

(57) **Abstract**

In a medical device, an antimicrobial agent is combined with an elastomeric polymer material. The antimicrobial agent includes at least one chlorhexidine/fatty acid salt which is a neutralization product of chlorhexidine base and a fatty acid having between 12 and 18 carbon atoms.

## Description

### FIELD OF THE INVENTION

The present invention generally relates to medical devices having antimicrobial properties. More particularly, the present invention pertains to elastomeric medical devices having antimicrobial properties and the optimization thereof.

### BACKGROUND OF THE INVENTION

It is generally known that in various medical procedures, the use of medical devices having antimicrobial properties may reduce the incidence of infection in the patient. Examples of such devices include catheters, grafts, stents, sutures, and the like. Typically, the antimicrobial agent used in conventional medical devices diffuses from the substrate of the device into the patient to create a zone of inhibition around the device. As the antimicrobial agent continues to diffuse away from the device, the concentration is maintained by the continued diffusion of the antimicrobial agent from the device. Once this reservoir is depleted, the concentration of antimicrobial agent may fall below an effective level and the device no longer retains the antimicrobial properties.

For relatively short procedures lasting less than a day, for example, conventional antimicrobial medical devices may retain the antimicrobial properties for the duration of the procedure. For example, silver compounds (e.g., silver chlorine and silver oxide), chlorhexidine, iodine, triclosan, and the like. These and other conventional antimicrobial agents rapidly diffuse out from the medical device to create a zone of inhibition that reduces bacterial contamination. Unfortunately, for longer duration procedures, the conventional medical device may need to be removed and a new device used to replace the spent device in order to retain the antimicrobial properties. For procedures lasting many days or weeks, this need for replacement may be burdensome.

Accordingly, it is desirable to provide an antimicrobial medical device capable of overcoming the disadvantages described herein at least to some extent.

### SUMMARY OF THE INVENTION

The foregoing needs are met, to a great extent, by the present invention, wherein in one respect an antimicrobial medical device and method of optimizing the antimicrobial properties thereof is provided.

An embodiment of the present invention pertains to a medical device including an antimicrobial agent in an elastomeric polymer material. The antimicrobial agent includes at least one chlorhexidine/fatty acid salt which is a neutralization product of chlorhexidine base and a fatty acid having between 12 and 18 carbon atoms.

Another embodiment of the present invention relates to a method of making an infection-resistant medical device. In this method, an antimicrobial agent comprising a chlorhexidine/fatty acid salt is selected. The chlorhexidine/fatty acid salt includes a neutralization product of chlorhexidine base and a fatty acid having between 12 and 18 carbon atoms. In addition, an elastomeric polymer material is combined with an effective amount of the antimicrobial agent.

Yet another embodiment of the present invention pertains to a medical catheter. The medical catheter includes an elongated hollow tube, an exterior surface of the elongated hollow tube having an elastomeric polymer, and a chlorhexidine/fatty acid salt being disposed in the elastomeric polymer. The chlorhexidine/fatty acid salt is a neutralization product of chlorhexidine base and a fatty acid having between 12 and 18 carbon atoms.

Yet another embodiment of the present invention pertains to a method of optimizing infection resistance in a medical device. In this method, a duration of use for the medical device is determined, an elastomeric polymer is selected to make the medical device, and a rate of release is determined for a set of chlorhexidine/fatty acid salts from the elastomeric polymer. The rate of release is determined for the duration of use. The set of chlorhexidine/fatty acid salts are a set of neutralization products of chlorhexidine base and one or more fatty acid having between 12 and 18 carbon atoms. In addition, a chlorhexidine/fatty acid salt is selected from the set of chlorhexidine/fatty acid salts having a respective rate of release exceeding a predetermined minimum rate of release for the duration of use.

Yet another embodiment of the present invention pertains to a method of optimizing infection resistance in a medical device. In this method, a duration of use for the medical device is determined, an elastomeric polymer is selected to make the medical device, a zone of inhibition is determined for a set of chlorhexidine/fatty acid salts from the elastomeric polymer. The zone of inhibition is determined for the duration of use. The set of chlorhexidine/fatty acid salts are a set of neutralization products of chlorhexidine base and one or more fatty acid having between 12 and 18 carbon atoms. In addition, a chlorhexidine/fatty acid salt is selected from the set of chlorhexidine/fatty acid salts having a respective zone of inhibition exceeding a predetermined minimum zone of inhibition for the duration of use.

There has thus been outlined, rather broadly, certain embodiments of the invention in order that the detailed description thereof herein may be better understood, and in order that the present contribution to the art may be better appreciated. There are, of course, additional embodiments of the invention that will be described below and which will form the subject matter of the claims appended hereto.

In this respect, before explaining at least one embodiment of the invention in detail, it is to be understood that the invention is not limited in its application to the details of construction and to the arrangements of the components set forth in the following description or illustrated in the drawings. The invention is capable of embodiments in addition to those described and of being practiced and carried out in various ways. Also, it is to be understood that the phraseology and terminology employed herein, as well as the abstract, are for the purpose of description and should not be regarded as limiting.

As such, those skilled in the art will appreciate that the conception upon which this disclosure is based may readily be utilized as a basis for the designing of other structures, methods and systems for carrying out the several purposes of the present invention. It is important, therefore, that the claims be regarded as including such equivalent constructions insofar as they do not depart from the spirit and scope of the present invention.

### BRIEF DESCRIPTION OF THE DRAWINGS

FIG. 1 is an example of a graph showing time in days (abscissa) of the percentage release of antimicrobial agent from a medical article (ordinate).

FIG. 2 is an example of a chart showing time in days (abscissa) verses a zone of inhibition in millimeters (ordinate).

FIG. 3 is a perspective view of a catheter suitable for use with an embodiment of the invention.

FIG. 4 is a cross sectional view of the catheter according to FIG. 3.

FIG. 5 is a detailed view of the catheter according to FIG. 3.

### DETAILED DESCRIPTION

Embodiments of the invention provide infection resistant medical devices and methods of optimizing the duration of the antimicrobial properties of medical devices. In a particular embodiment, straight chain fatty acids, such as for example dodecanoic, myristic, palmitic and stearic acid, are utilized to make a chlorhexidine/fatty acid salt. These chlorexidines salts include, respectively: chlorhexidine laurate, chlorhexidine myristate, chlorhexidine palmitate and chlorhexidine stearate. Throughout the text of this disclosure it should be understood that the term chlorhexidine laurate will be used as a substitute for chlorhexidine dilaurate as will chlorhexidine myristate, chlorhexidine palmitate, chlorhexidine stearate and chlorhexidine acetate be used as substitutes for chlorhexidine dimyristate, chlorhexidine dipalmitate, chlorhexidine distearate and chlorhexidine diacetate respectively.

One or more of these chlorhexidine/fatty acid salts can be combined with any suitable medical device to produce an infection resistant or antimicrobial medical device. Examples of suitable medical devices include catheters, grafts, stents, sutures, and the like. More particularly, suitable medical devices include an elastomeric material such as, for example, polyurethanes, silicones, nitrile rubber, fluoroelastomers, polyolephin elastomers, latex-type elastomers, and the like. In addition to synthetic elastomers, various natural elastomers such as those made from collagen, elastin, cellulose, proteins, carbohydrates, etc. are also suitable for use with embodiments of the invention. A specific example of a suitable elastomer includes Tecoflex® thermoplastic polyurethane (TPU) and other elastomers and copolymers manufactured by The Lubrizol Corp., Wickliffe, OH 44092, U.S.A. It is an advantage of various embodiments of devices made according to the present invention, that infection resistant medical devices may be imbued with antimicrobial properties over an extended period of days, weeks, etc. Methods of treating these medical articles include, but are not limited to, dip surface coating, soaking (bulk impregnation), compounding and extrusion, or any suitable hot melt process.

In an embodiment of the present invention, a specific chain length range of fatty acids is utilized to synthesize a chlorhexidine/fatty acid salt to produce an optimal antimicrobial activity over the duration of use of the medical article, which can be as long as several days to weeks. For example, to provide effective anti-microbial activity for a duration of two or more days to about several weeks in aqueous media, the straight chain fatty acid used to synthesize the chlorhexidine/fatty acid salt preferably contains between 10 and 18 carbons, and more preferably between 12 and 16 carbons. In general, the shorter the fatty acid chain, the faster the diffusion rate and the longer the fatty acid chain length, the slower the diffusion rate. This general property remains true regardless of the materials of the medical article, however, different materials may have different relative rates of diffusion. For example, relatively high density or highly cross linked elastomers may slow the diffusion rate of the chlorhexidine/fatty acid salt. Accordingly, using fatty acids containing less than 12 carbons produces a chlorhexidine/fatty acid salt that may exhibit an unacceptably rapid release from a treated medical article that includes a low density elastomer. Conversely, using fatty acids containing a chain of 18 or more carbons produces a chlorhexidine/fatty acid salt that may exhibit an unacceptably slow release from a relatively high density elastomer. Therefore, optimization may be achieved by combining one or more particular chlorhexidine/fatty acid salts with a particular elastomer.

In general, to optimize the infection resistance in a medical article or device, a duration of intended use for the medical device is determined and an elastomeric polymer is selected to make the medical device. A variety of chlorhexidine/fatty acid salts are synthesized to generate a set of chlorhexidine/fatty acid salts. According to assays described herein, the rate of release from an elastomer differs as a function of the length of the carbon chain in the fatty acid. The respective rate of release for each chlorhexidine/fatty acid salt from the elastomer over the duration of intended use is determined. Based on the rate of release over the duration of intended use, a particular chlorhexidine/fatty acid salt is selected from the set of chlorhexidine/fatty acid salts.

Another method of optimizing the infection resistance of a medical article is to perform a zone of inhibition assay as described herein on the set of chlorhexidine/fatty acid salts. The results of the zone of inhibition assay may be utilized to determine the most effective chlorhexidine/fatty acid salt for the particular elastomer.

As described herein, experiments were performed to synthesize the target chlorhexidine/fatty acid salts, these chlorheidine salts were infused into various elastomers, and the duration of antimicrobial activity was determined. Specifically, chlorhexidine laurate, chlorhexidine myristate, chlorhexidine palmitate and chlorhexidine stearate were synthesized by reacting chlorhexidine base with: lauric acid, a 12-carbon chain fatty acid; myristic acid, a 14-carbon chain fatty acid; palmitic acid, a 16-carbon chain fatty acid; and stearic acid, an 18-carbon chain fatty acid, respectively.

Polymer based dipping solutions were made with each fatty acid salt and chlorhexidine diacetate, synthesized from chlorhexidine base and acetic acid. Polyurethane tubes, containing a colorant, were dip coated with the polymer/salt solution. The rate of release of each salt from the polymeric article in water was evaluated for a period of 4 weeks. In addition, the antimicrobial activity of the polymeric article was evaluated. In these experiments, the adherence of challenge organisms: gram positive, gram negative and yeast, to the polymeric articles, after release of the anti-microbial agent, was investigated. It was surprisingly found that the chlorhexidine diacetate, was not able to provide an antimicrobial effect against *Pseudomonas aeruginosa* after 7 days. This observation may be due to the relatively rapid diffusion of chlorheidine diacetate from the particular elastomer tested. It was also observed that chlorhexidine stearate did not provide an antimicrobial effect beyond the first day. In this experiment, the diffusion rate of chlorhexidine stearate may be so low given the particular elastomer tested, that the concentration of chlorhexidine stearate in the vicinity of the medical article may have been below the level required to inhibit adherence.

In another experiment, a polyurethane foam was impregnated with the chlorhexidine/fatty acid salts previously mentioned along with chlorhexidine caprate (synthesized from chlorhexidine base and capric acid, a 10-carbon fatty acid). The antimicrobial activities of these foams, which could be used as dressings or devices to cushion or fill other voids, were investigated by the time course of the radii of their zones of inhibition against challenge organisms including yeasts. It was observed that the foams impregnated with chlorhexidine acetate, chlorhexidine caprate or chlorhexidine stearate produced zones of inhibition that were smaller than those produced by the foams impregnated with chlorhexidine laurate, chlorhexidine myristate or chlorhexidine palmitate after several daily transfers.

Of note, while polymer based dipping and soaking are specifically described herein, in other embodiments the chlorhexidine/fatty acid salt may be combined with the elastomer via melt compounding, extrusion/molding, spray coat, dip coat, spin coat, impregnation, surface attached, complexed, immobilized, and/or the like. The chlorhexidine/fatty acid salt may be present in a coating, evenly distributed throughout the medical article or elastomeric component thereof, may be present in a plurality of laminated layers, in discrete patches or zones, and the like. In addition, the chlorhexidine/fatty acid salts may be present as liquids, suspensions, as crystalline and/or amorphous solids, as nanoparticles or micelles, emulsions, microemulsions, microspheres, liposomes, and/or the like.

A significant benefit of various embodiments of the invention is the use of a medical device for a prolonged period without a significant increase of the chlorhexidine exposure to the patient. According to these and other embodiments, in addition to the chlorhexidine/fatty acid salt, the medical article may include any suitable additional agent. Suitable agents include additional antimicrobial agents, antiseptics, chemotherapeutics, antimicrobial peptides, mimetics, antithrombogenic, fibrinolytic, anti-inflammatory, antipain, antinausea, vasodilators, antiproliferatives, antifibrotics, growth factors, cytokines, and/or the like.

### METHODS AND RESULTS

### EXAMPLE 1:

To synthesize the various chlorhexidine/fatty acid salts, 2.1 molar equivalent of capric acid (decanoic acid, Sigma-Aldrich), lauric acid (dodecanoic acid, Sigma-Aldrich), myristic acid (tetradecanoic acid, Sigma-Aldrich), palmitic acid (hexadecanoic acid, Sigma-Aldrich), and stearic acid (octadecanoic acid, Sigma-Aldrich), were added to individual slurries of 15.1g chlorhexidine base (Arrow International, Reading, PA) in 150ml of alcohol (Fisher Scientific). Solution went clear and then precipitation occurred. Precipitate was rinsed with 100ml alcohol and filtered twice, after which it was vacuumed dried at 25°C for 24hrs.

**Table 1: % recovery of precipitated chlorhexidine/fatty acid salt**

| **Sample ID** | **% recovery** |
|---|---|
| Chlorhexidine Caprate | 74.0 |
| Chlorhexidine Laurate | 88.7 |
| Chlorhexidine Myristate | 98.1 |
| Chlorhexidine Palmitate | 92.4 |
| Chlorhexidine Stearate | 97.2 |

### EXAMPLE 2:

A solution of each respective chlorhexidine/fatty acid salt was prepared and used to infuse a sample of elastomeric material. To perform the test, individual samples, 8cm pieces of polyurethane tube containing a colorant, were surface dip coated in respective solutions of the chlorhexidine/fatty acid salts. The solutions were prepared as follows: 8.6g of Tecoflex (Noveon, Cleveland, OH) was dissolved in 174g of Tetrahydrofuran and methanol. 5.88g of chlorhexidine acetate, chlorhexidine laurate, chlorhexidine myristate, chlorhexidine palmitate and chlorhexidine stearate were individually dissolved into the polymer-solvent mixture. The samples were immersed into the solvent-polymer-salt mixture and then dried at room temperature, evaporating the solvent. A polymeric coating was produced on the surface of the tubes. The amounts of chlorhexidine/fatty acid salt adhered per cm of the tube was measured (Table 2) by exhaustively extracting the salt and measuring UV absorbance at 253 nm relative to controls:

**Table 2: chlorhexidine/fatty acid salt introduced to medical article**

| **Salt coated onto article** | **Salt content (ug/cm)** |
|---|---|
| Chlorhexidine Acetate | 225 |
| Chlorhexidine Laurate | 189 |
| Chlorhexidine Myristate | 203 |
| Chlorhexidine Palmitate | 227 |
| Chlorhexidine Stearate | 222 |

As shown in Table 2, each sample retained approximately 200 µg/cm with chlorhexidine laurate retaining the relative least at 189 µg/cm and chlorhexidine palmitate retaining the relative most at 227 µg/cm. The values from Table 2 were used to determine the initial content of chlorhexidine/fatty acid salt used in subsequent calculations.

### EXAMPLE 3:

For each of the samples, the rate of release of chlorhexidine/fatty acid salts into an aqueous solution was determined. To perform the rate of release test, 1 cm from each of the coated polyurethane tubes, were submerged in DeIonized (DI) water and incubated at 37°C for a period of 28 days to determine the rate of release of the salts from the respective 1 cm samples of coated polyurethane tubing. The water was collected and replaced with fresh water after approximately 2 hours. The water was again collected and replaced with fresh water after the first day and every seven days thereafter. The amount of chlorhexidine/fatty acid salt in the collected water was measured with an Agilent 8453 UV-Vis Diode Array Detector with a manual setup using a cuvette w/ path length of 1cm. Chlorhexidine absorbance was read at 253nm and concentration was calculated via a calibration curve developed using standards of 1, 5, 10, 20, 30, 35, and 40 µg/mL chlorhexidine acetate in DI Water.

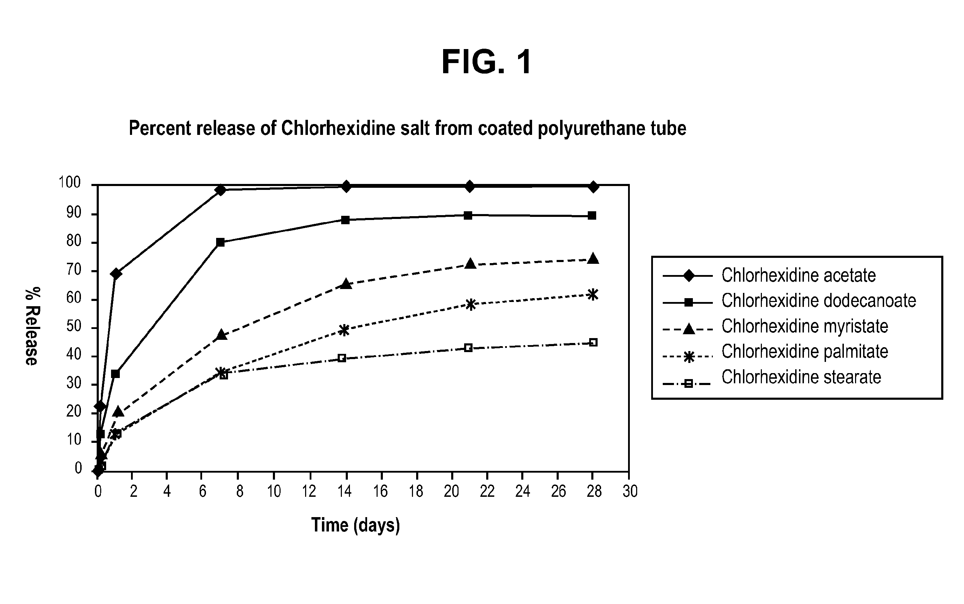

The percentage of chlorhexidine/fatty acid salt released from the sample into an aqueous media (percent release) was calculated according to the following equation: Percent release = (cumulative release at that time period / initial content) * 100. The results are presented in Table 3, with a graphical representation of percent released shown in FIG. 1.

**Table 3: Cumulative release (•g) of the chlorhexidine/fatty acid salts from the coated polyurethane tubes**

| | **Time (Days)** | | | | |
|---|---|---|---|---|---|
| **Salt coated onto article** | **1** | **7** | **14** | **21** | **28** |
| Chlorhexidine Acetate | 155 | 222 | 224 | 224 | 224 |
| Chlorhexidine Laurate | 62 | 145 | 165 | 167 | 167 |
| Chlorhexidine Myristate | 40 | 98 | 133 | 147 | 151 |
| Chlorhexidine Palmitate | 28 | 79 | 112 | 132 | 140 |
| Chlorhexidine Stearate | 30 | 76 | 87 | 95 | 99 |

As shown in Table 3, approximately all of the chlorhexidine acetate was released from the coated sample within 7 days. By day 14, approximately all of the chlorhexidine laurate was released from the coated sample. In contrast, both chlorhexidine myristate and chlorhexidine palmitate were released from the coated sample at 4 µg and 8 µg over the 7 day period from 21 to 28 days.

FIG. 1 is an example of a graph showing time in days (abscissa) verses a percentage release of an antimicrobial agent from a medical article (ordinate). As shown in FIG. 1, each of the different chlorhexidine/fatty acid salts exhibits release characteristics from the coated samples that differ from the release characteristics of the other chlorhexidine/fatty acid salts. In general, the shorter the fatty acid carbon chain, the more rapid the release into the aqueous surroundings from the coated samples. Most notably, the control chlorhexidine acetate was released from the coated sample at approximately 70% of the initial salt concentration within 1 day. In contrast, chlorhexidine caprate (dodecanoate) was released from the coated sample at 70% of the initial salt concentration only after approximately 7 days and the coated sample released chlorhexidine stearate at only approximately 40% of the initial salt concentration after 28 days. Of note, FIG. 1 further shows that both chlorhexidine myristate and chlorhexidine palmitate are continually released at an appreciable rate throughout the duration of the test.

### EXAMPLE 4:

The antimicrobial effectiveness of the coated samples was subjected to different durations of DI water extraction by basic broth adherence assay. For each chlorhexidine/fatty acid salt tested, 3 samples of coated tubing were prepared and assayed. To prepare the inoculum, a culture of *Pseudomonas aeruginosa* ATCC 27853 was used to challenge the coated polyurethane tubes and controls. The negative control used was a length of uncoated polyurethane tube. The challenge microorganism suspension was prepared as follows: approximately three colonies were removed from a secondary working culture plated on Trypticase Soy Agar (TSA) (BD-Falcon) with 5% sheep's blood and added to 10ml of Trypticase Soy Broth (TSB). The vial was vortexed for approximately 30 seconds and incubated for 4 hours at 37°C at 100 rpm. Following incubation, the vial was removed and vortexed. The optical density of the organism suspension was read. The inoculum was then diluted in TSB to a concentration of 5 x 104 colony forming units per milliliter (CFU/mL). This concentration was confirmed by the Miles and Misra drop count method.

The basic broth adherence assay was performed in a 96 well micro titer plate. To each well of the micro titer plate containing either a test article or a control, 900µl of TSB was added. To this, 100µl of the adjusted inoculum was added (Note: Due to the 1/10 dilution per well, the starting inoculum concentration of each organism was 103 CFU/mL for *Pseudomonas aeruginosa*). The micro titer plates were then sealed via parafilm® around the edges and wrapped in foil to avoid evaporation of liquid and light contact with the samples. The plates were then placed inside a shaker incubator set at 37°C and 100 rpm for 24 hours. Incubation at 37°C then continued until the elapse of 7 days, 14 days, and 21 days, respectively, for each of the 3 samples prepared for each chlorhexidine/fatty acid salt.

At the completion of the respective incubation periods, the samples were rinsed, sonicated, the sonicate was plated and adherence was evaluated. More particularly, following incubation, all samples and controls were dip-rinsed twice in phosphate buffered saline (PBS) to remove the surrounding media. After rinsing, test articles were sonicated in micro titer plate wells containing 1 mL of Dey-Engley (DE) neutralizing broth (Difco). The sonication liquid was then serially diluted, and plated onto the surface of DE neutralizing agar. Plates were incubated at 37°C for 24 to 48 hours and colonies were counted. Recovery counts were recorded as CFU/mL. The results are presented in Table 4:

**Table 4: Log₁₀ reduction of adhered Pseudomonas aeruginosa onto the surface of coated polyurethane tubes**

| | **Time (Days)** | | |
|---|---|---|---|
| **Salt coated onto article** | **7** | **14** | **21** |
| Chlorhexidine Acetate | 5 | 0 | 0 |
| Chlorhexidine Laurate | 7 | 0 | 0 |
| Chlorhexidine Myristate | 7 | - | 7 |
| Chlorhexidine Palmitate | 7 | 7 | 7 |
| Chlorhexidine Stearate | 0 | 0 | 0 |

As shown in Table 4, as compared to the control sample, the chlorhexidine stearate coated sample did not inhibit adherence of the challenge organism. Both chlorhexidine acetate and chlorhexidine laurate initially inhibited adherence of the challenge organism but that inhibition was not apparent at 14 days or thereafter. However, both chlorhexidine myristate and chlorhexidine palmitate were observed to decrease the adherence of the challenge organism by seven orders of magnitude for the full duration (21 days) of the assay.

### EXAMPLE 5:

To evaluate antimicrobial properties of polyurethane foam samples infused with the various chlorhexidine/fatty acid salts, a zone of inhibition assay was performed. In this assay, 1.4% (w/v) of chlorhexidine acetate, chlorhexidine caprate, chlorhexidine laurate, chlorhexidine myristate, chlorhexidine palmitate and chlorhexidine stearate solutions in methanol were prepared. A supply of 8mm discs were punched out of a sheet of polyurethane foam (Rynel medical grade polyurethane foam) with biopsy punches (Miltex Kai). Several discs were placed in each of six 50ml centrifuge tubes. Approximately 5ml of each of the different methanol/chlorhexidine/fatty acid salt solutions was added to each respective tube. The discs were centrifuged for 5 minutes to remove any air bubbles and soaked for 1hr to infuse the foam discs with the respective chlorhexidine/fatty acid salt. Infused discs were transferred to clean centrifuge tubes and vacuum dried at 25°C for 24hrs to remove any residual solvents. Initial concentrations of salts, impregnated into the punches, were determined by dissolving the impregnated foams and by high pressure liquid chromatography (HPLC) analysis using an Agilent Eclipse XDB-CN 3u 4.6x150mm column with initial mobile phase = 65% DI water/0.2% trifluoroacetic acid (TFA): 35% Acetonitrile/0.2%TFA. Chlorhexidine absorbance was read at 253nm and concentration was calculated via a chlorhexidine calibration curve. The amount of salts impregnated into the 8mm discs are listed in table 5;

**Table 5: chlorhexidine/fatty acid salt content in medical article**

| **Salt impregnated into foam** | **Salt content (mg)** |
|---|---|
| Chlorhexidine Acetate | 5.5 |
| Chlorhexidine Caprate | 5.8 |
| Chlorhexidine Laurate | 6.0 |
| Chlorhexidine Myristate | 4.4 |
| Chlorhexidine Palmitate | 4.0 |
| Chlorhexidine Stearate | 4.9 |

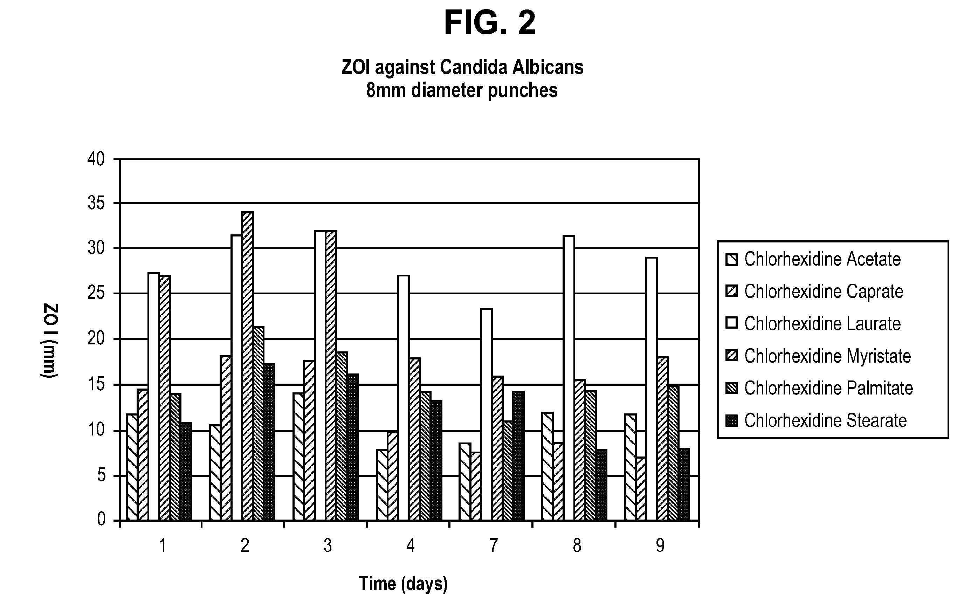

As shown in Table 5, the discs retained approximately 4 to 6 mg of the chlorhexidine/fatty acid salt. To perform the zone of inhibition assay on the impregnated polyurethane foam discs, agar plates were prepared by mixing11g Mueller-Hinton cation-adjusted broth and 1.5g agarose in 500ml DI water. The agar was autoclaved for 15 minutes at 121°C, 15 psi. 10ml of the still hot, liquid agar was pipetted onto petri dishes. The challenge organism, *Candida albicans* ATCC 10231, was cultivated in Trypticase soy broth, standard microbiological growth medium (TSB). Sterile cotton swabs were dipped into the undiluted culture and the plated agar was fully inoculated with swabs. The 8mm polyurethane foam discs were transferred daily to a freshly inoculated agar for 9 days. The zone of inhibition (ZOI) was measured using calipers. Actual ZOI was measured by subtracting the diameter of the foam (when wet) from the zone measured and dividing by 2. The results are presented in Table 6, with a graphical representation of the ZOI in FIG. 2;

**Table 6: ZOI (mm) produced by impregnated polyurethane foam punches against Candida albicans**

| **Salt impregnated into foam** | **Time (Days)** | | | | | | |
|---|---|---|---|---|---|---|---|
| | **1** | **2** | **3** | **4** | **7** | **8** | **9** |
| Chlorhexidine Acetate | 12 | 11 | 14 | 8 | 8 | 12 | 12 |
| Chlorhexidine Caprate | 14 | 18 | 18 | 10 | 7 | 9 | 7 |
| Chlorhexidine Laurate | 27 | 31 | 32 | 27 | 23 | 31 | 29 |
| Chlorhexidine Myristate | 27 | 34 | 32 | 18 | 16 | 16 | 18 |
| Chlorhexidine Palmitate | 14 | 21 | 19 | 14 | 11 | 14 | 15 |
| Chlorhexidine Stearate | 11 | 17 | 16 | 13 | 14 | 8 | 8 |

FIG. 2 is an example of a graph showing time in days (abscissa) verses a zone of inhibition in millimeters (ordinate) against *Candida albicans* for various different chlorhexidine salts. As shown in FIG. 2, over the 9 day assay, chlorhexidine laurate exhibits the relatively largest ZOI in comparison to the other chlorhexidine/fatty acid salts.

Therefore, as shown herein, the material characteristics, such as composition and form, of the medical article may affect the release rate of a chlorhexidine antimicrobial agent combined therein. To optimize the release rate of the chlorhexidine, it may be combined with fatty acids having a carbon chain length of 12 to 18 carbons. The release rate and/or ZOI of these entities may be tested in the medical article to determine the best fit given a predetermined duration of use for the medical device. It has thus been shown that by performing syntheses and assays as described herein and with proper selection of material and antimicrobial agent, infection resistant medical devices may be fabricated that possess antimicrobial properties over an extended period of days, weeks, etc.

### EXAMPLE 6:

To evaluate the incorporation of fatty acid salts of chlorhexidine in silicone elastomers, incorporation of the solvent without fatty acid salts of chlorhexidine was initially determined for the silicone tubing. The swelling of silicone tubing in tetrahydrofuran (THF) and THF + methanol mixtures was measured by weighing a piece of silicone tubing and then immersing it in an excess of solvent for varying time durations. For each solvent mixture and duration, the respective swollen piece of silicone tubing was removed, blotted dry and reweighed. Mass % swelling (defined as 100 * [swollen weight-dry weight]) was calculated and is reported below:

**Table 7: solvent incorporation in silicone tubing**

| **Soak Time (min)** | **THF (%)** | **Methanol (%)** | **% Swell** |
|---|---|---|---|
| 20 | 90 | 10 | 83 |
| 30 | 90 | 10 | 93 |
| 60 | 90 | 10 | 125 |
| 120 | 90 | 10 | 116 |
| 1 day | 90 | 10 | 125 |
| | | | |
| 60 | 80 | 20 | 93 |
| 120 | 80 | 20 | 96 |
| 1 day | 80 | 20 | 102 |
| | | | |
| 60 | 60 | 40 | 52 |
| 120 | 60 | 40 | 54 |
| 1 day | 60 | 40 | 50 |

Chlorhexidine diacetate, Chlorhexidine dodecanoate and chlorhexidine palmitate were loaded into silicone tubing by first dissolving the chlorhexidine salt in a 90% THF + 10% Methanol solution. The tubing was then immersed in this solution for one day where it swelled and chlorhexidine salt diffused into the polymer matrix. The impregnated piece of tubing was removed and then vacuum dried to remove all volatile solvent.

Impregnated chlorhexidine was extracted from the tubing by immersing in an excess of THF to fully swell it and then adding an equal volume of water to extract the chlorhexidine salt. The quantity of chlorhexidine was determined by previously enumerated HPLC method. It is reported below for different concentration impregnation solutions in terms of the equivalent mass of chlorhexidine base extracted:

**Table 8: chlorhexidine/fatty acid salt content in silicone elastomeric medical article**

| **Sample** | **Salt content (µg/cm)** |
|---|---|
| 1% Chlorhexidine diacetate | 262 |
| 3% Chlorhexidine diacetate | 395 |
| 6% Chlorhexidine diacetate | 374 |
| 10% Chlorhexidine diacetate | 439 |
| | |
| 1% Chlorhexidine dodecanoate | 249 |
| 3% Chlorhexidine dodecanoate | 408 |
| 6% Chlorhexidine dodecanoate | 526 |
| 10% Chlorhexidine dodecanoate | 614 |
| | |
| 1% Chlorhexidine palmitate | 133 |
| 3% Chlorhexidine palmitate | 205 |
| 6% Chlorhexidine palmitate | 356 |
| 10% Chlorhexidine palmitate | 366 |
| 20% Chlorhexidine palmitate | 407 |

Surprisingly CHDD was absorbed at much higher concentrations than the other salts and CHP was soluble at higher concentrations in the swelling impregnation solvent mixture. None of the chlorhexidine salts was appreciably soluble in pure THF.

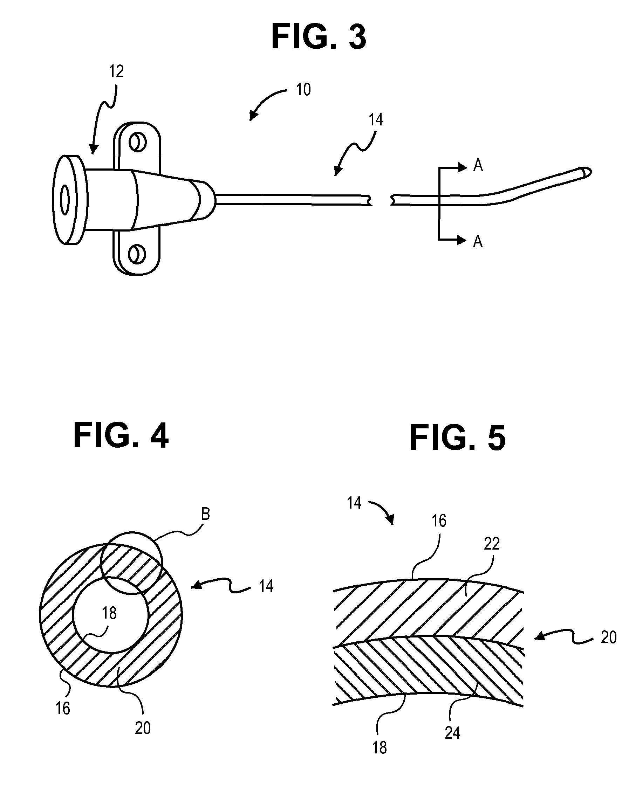

FIG. 3 is a perspective view of a catheter 10 suitable for use with an embodiment of the invention. As shown in FIG. 3, the catheter 10 includes a catheter hub 12 and catheter body 14. As is generally known, the catheter body 14 may be inserted into a patient and the catheter 10 may be accessed via the catheter hub 12. In a particular embodiment, the catheter body 14 includes one or more chlorhexidine/fatty acid salts described herein. It is an advantage of this and other embodiments that the catheter may remain in the patient for an extended period relative to conventional catheters. However, it is to be understood that the various embodiments of the invention are not limited to catheters. Instead, the various embodiments may include any suitable elastomeric or elastomeric containing medical device. Specific examples of suitable devices includes: lens; contact lens; intraocular lens; intraocular implant; intracochlear implant; tube; stent; mask; suture; string; yarn; woven or non-woven mesh; shunt; hydrocephalous shunt; orthopedic devices (coating); fleece or felt; dressing; catheter; intrauterine device (IUD); ring; vaginal ring; cartilage implant; testicular implant; breast implant; sponge; foam; facial implant; valve; sphincter; dental implant; periodontal implant; penile implant; membrane; patch; tendon; ligament; joint; nail; rod; pin; screw; bolt; tape; cable; tissue or muscle bulking agent; wrap; graft; particle(s); and the like.

FIG. 4 is a cross sectional view A-A of the catheter body 14 according to FIG. 3. As shown in FIG. 4, the catheter body 14 includes an outer surface 16, inner surface 18, and wall 20. According to various embodiments, the chlorhexidine/fatty acid salt may be included in one or more of the outer surface 16, inner surface 18, and wall 20.

FIG. 5 is a detailed view B of the catheter body 14 according to FIG. 3. As shown in FIG. 5, the catheter body 14 may include an outer wall 22 and inner wall 24. In a particular example, the outer wall 22 may include an elastomeric material suitable for use with one or more of the various chlorhexidine/fatty acid salts described herein. The inner wall 24 may variously include an elastomeric or non-elastomeric material. For example, the inner wall 24 may include a vinyl polymer or metal such as stainless steel. In addition, while two layers are shown, in other embodiments 3 or more layers may be included.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A medical device comprising an antimicrobial agent in an elastomeric polymer material, the antimicrobial agent including at least one chlorhexidine/fatty acid salt which is a neutralization product of chlorhexidine base and a fatty acid having between 12 and 18 carbon atoms.

2. The medical device according to claim 1, wherein the fatty acid comprises between 12 and 16 carbon atoms.

3. The medical device according to claim 1 or 2, wherein the chlorhexidine/fatty acid salt comprises one of the following: a chlorhexidine Laurate (chlorhexidine dodecanoate), a chlorhexidine Myristate (chlorhexidine tetradecanoate), a chlorhexidine Palmitate (chlorhexidine hexadecanoate) or a chlorhexidine Stearate (chlorhexidine octadecanoate).

4. The medical device according to claim 1, wherein the antimicrobial agent further comprises:
a mixture comprising a plurality of chlorhexidine/fatty acid salts, wherein each chlorhexidine/fatty acid salt of the plurality of chlorhexidine/fatty acid salts comprises a fatty acid having between 12 and 18 carbon atoms.

5. The medical device according to claim 1, wherein the elastomeric polymer comprises one of : polyurethane, a polyurethane foam, a silicone, a fluoroelastomer, a polyolephin elastomer, a latex-type elastomer.

6. A method of making an infection-resistant medical device, the method comprising:
selecting an antimicrobial agent comprising a chlorhexidine/fatty acid salt, wherein the chlorhexidine/fatty acid salt comprises a neutralization product of chlorhexidine base and a fatty acid having between 12 and 18 carbon atoms; and
combining an elastomeric polymer material with an effective amount of the antimicrobial agent.

7. The method according to claim 6, wherein the step of selecting further comprises: determining a diffusion rate of the antimicrobial agent from the elastomeric polymer material.

8. The method according to claim 6, wherein the step of selecting further comprises: synthesizing the chlorhexidine/fatty acid salt in a neutralization reaction with the chlorhexidine base and the fatty acid having between 12 and 18 carbon atoms, for example between 12 and 16 carbon atoms.

9. The method according to claim 8, wherein the step of synthesizing further comprises reacting the chlorhexidine base with one of: a Stearic acid (octadecanoic acid) to synthesize a chlorhexidine Stearate (chlorhexidine octadecanoate), a Lauric acid (dodecanoic acid) to synthesize a chlorhexidine Laurate (chlorhexidine dodecanoate), a Myristic acid (tetradecanoic acid) to synthesize a chlorhexidine Myristate (chlorhexidine tetradecanoate) or a Palmitic acid (hexadecanoic acid) to synthesize a chlorhexidine Palmitate (chlorhexidine hexadecanoate).

10. The method according to claim 6, wherein the step of selecting further comprises: selecting a mixture of antimicrobial agents comprising a plurality of chlorhexidine/fatty acid salts, wherein each chlorhexidine/fatty acid salt of the plurality of chlorhexidine/fatty acid salts comprises a fatty acid having between 12 and 18 carbon atoms.

11. The method according to claim 6, further comprising the step of one of the following:
selecting a polyurethane to fabricate the elastomeric polymer material;
selecting a polyurethane foam to fabricate the elastomeric polymer material;
selecting a silicone to fabricate the elastomeric polymer material;
selecting a fluoroelastomer to fabricate the elastomeric polymer material;
selecting a polyolephin elastomer to fabricate the elastomeric polymer material; or
selecting a latex-type elastomer to fabricate the elastomeric polymer material.

12. The method according to claim 6, wherein the step of combining further comprises: soaking the elastomeric polymer material in a solution of the antimicrobial agent.

13. A medical catheter comprising:
an elongated hollow tube;
an exterior surface of the elongated hollow tube including an elastomeric polymer; and a chlorhexidine/fatty acid salt being disposed in the elastomeric polymer, wherein the chlorhexidine/fatty acid salt is a neutralization product of chlorhexidine base and a fatty acid having between 12 and 18 carbon atoms.

14. A method of optimizing infection resistance in a medical device, the method comprising:
determining a duration of use for the medical device;
selecting an elastomeric polymer to make the medical device;
determining a rate of release for a set of chlorhexidine/fatty acid salts from the elastomeric polymer, the rate of release being determined for the duration of use, the set of chlorhexidine/fatty acid salts being a set of neutralization products of chlorhexidine base and one or more fatty acid having between 12 and 18 carbon atoms; and
selecting a chlorhexidine/fatty acid salt from the set of chlorhexidine/fatty acid salts having a respective rate of release exceeding a predetermined minimum rate of release for the duration of use.

15. A method of optimizing infection resistance in a medical device, the method comprising:
determining a duration of use for the medical device;
selecting an elastomeric polymer to make the medical device;
determining a zone of inhibition for a set of chlorhexidine/fatty acid salts from the elastomeric polymer, the zone of inhibition being determined for the duration of use, the set of chlorhexidine/fatty acid salts being a set of neutralization products of chlorhexidine base and one or more fatty acid having between 12 and 18 carbon atoms; and
selecting a chlorhexidine/fatty acid salt from the set of chlorhexidine/fatty acid salts having a respective zone of inhibition exceeding a predetermined minimum zone of inhibition for the duration of use.

16. A medical device or method according to any preceding claim wherein the fatty acid comprises a straight chain fatty acid.
